(19) Europäisches Patentamt
European Patent Office
Office européen des brevets

(11) Veröffentlichungsnummer: **0 040 741**
**B1**

(12) # EUROPÄISCHE PATENTSCHRIFT

(45) Veröffentlichungstag der Patentschrift: **08.06.83**

(51) Int. Cl.³: **C 07 C 68/02**, C 07 C 69/96, C 07 C 67/14, B 01 J 31/24

(21) Anmeldenummer: **81103580.7**

(22) Anmeldetag: **11.05.81**

(54) **Verfahren zur Herstellung von Chlorameisensäurearylestern.**

(30) Priorität: **22.05.80 DE 3019526**

(43) Veröffentlichungstag der Anmeldung: **02.12.81 Patentblatt 81/48**

(45) Bekanntmachung des Hinweises auf die Patenterteilung: **08.06.83 Patentblatt 83/23**

(84) Benannte Vertragsstaaten: **CH DE FR GB LI**

(56) Entgegenhaltungen:
**DE-A-2 804 227**
**US-A-3 170 946**

**Derwent Japanese Patents Report, Band 6, Nr. 13, 1967, London General Organic, Seite 2, Spalte 1**

**Derwent Japanese Patents Report, Band 6, Nr. 45, 1967, London General Organic, Seite 9, Spalte 1**

(73) Patentinhaber: **BAYER AG, Zentralbereich Patente, Marken und Lizenzen, D-5090 Leverkusen 1, Bayerwerk (DE)**

(72) Erfinder: **Rauchschwalbe, Günter, Dr., Hofstrasse 28, D-5000 Köln 80 (DE)**
Erfinder: **Blank, Heinz Ulrich, Dr., Am Geusfelde 35, D-5068 Odenthal (DE)**
Erfinder: **Mannes, Karl, Dr., Kurt-Schumacher-Ring 119, D-5090 Leverkusen (DE)**
Erfinder: **Dietmar, Mayer, Dr., Hamberger Strasse 51, D-5090 Leverkusen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Verfahren zur Herstellung von Chlorameisensäurearylestern

Die Erfindung betrifft ein Verfahren zur Herstellung von Chlorameisensäurearylestern durch Umsetzung von Phenolen mit Phosgen in Gegenwart von organischen Phosphorverbindungen.

Es ist bereits bekannt, Alkohole oder Phenole mit Phosgen zu den entsprechenden Chlorameisensäureestern umzusetzen (Ullmanns Enzyklopädie der technischen Chemie, 4. Auflage Band 9, Seite 381, Verlag Chemie, 1975). Während aliphatische Alkohole auch ohne Zusätze mit Phosgen reagieren können, müssen bei der Umsetzung von Phosgen mit Phenolen Zusätze angewendet werden, beispielsweise solche, die in der Lage sind, die freiwerdende Salzsäure zu binden.

So können beispielsweise anorganische Basen, wie wässrige Natronlauge, eingesetzt werden (DE-AS 1 117 598, GB 1 200 768). Diese Basen müssen in wenigstens stöchiometrischer Menge eingesetzt werden, was die Wirtschaftlichkeit des Verfahrens belastet und Probleme der Entsorgung aufwirft, da wenigstens stöchiometrische Mengen an anorganischen Salzen entstehen. Weiterhin beeinträchtigt die Arbeitsweise im Zweiphasensystem Wasser/organisches Lösungsmittel die Raumausbeute des Verfahrens.

Weiterhin ist der Zusatz von organischen Stickstoffbasen bekannt (DE-AS 1 213 419, US 3 211 776). Ihre Verwendung macht es jedoch nötig, die Reaktion in einem organischen Lösungsmittel oder unter erhöhtem Druck im Autoklaven durchzuführen. Auch hierdurch wird die Raumausbeute des Verfahrens belastet, bzw. werden Apparaturen zum Arbeiten unter Druck erforderlich. Das aus der zugesetzten Aminbase und dem freiwerdenden Chlorwasserstoff entstehende Hydrochlorid muss aus dem Reaktionsgemisch entfernt werden, beispielsweise durch Waschen der Lösung, Extraktion, Filtration oder Dekantieren, da anderenfalls das entstandene Hydrochlorid bei der folgenden Destillation die Apparatur verstopfen kann und zu Störungen führt. Zur Vermeidung dieser Komplikationen kann in Gegenwart eines Harzes, das Aminogruppen enthält, gearbeitet werden (US 3 211 775), jedoch muss auch dann unter Druck gearbeitet werden. Die zusätzlichen Kosten für diese polymeren Katalysatoren belasten die Wirtschaftlichkeit des Verfahrens. Weiterhin ist das Arbeiten mit Phosgen und dem entstehenden Chlorwasserstoff unter Druck aufwendig in bezug auf die Korrosion und die Aufrechterhaltung der Arbeitssicherheit.

Weiterhin begünstigen quartäre Ammoniumsalze die Reaktion von Phosgen mit Phenolen (US 3 255 230). Auch hierbei ist das Arbeiten in einem Lösungsmittel erforderlich. Gute Ausbeuten werden hierbei mit Ammoniumsalzen erzielt, die langkettige aliphatische Substituenten tragen, wie Stearyltrimethylammoniumchlorid (Chem. and Ind. 1965, 791 bis 793). Die Abtrennung dieser Zusätze erfolgt durch Filtration oder Chromatographie, was ebenso wie die speziellen Zusätze zu höheren Kosten führt.

Weiterhin wurden auch Carbonsäureamide, wie Dimethylformamid, als katalytisch wirksame Zusätze empfohlen (DE-AS 2 131 555; US 3 211 774). Dimethylformamid bildet jedoch mit Phosgen bei der Herstellung von Chlorameisensäurephenylester N,N-Dimethylcarbamoylchlorid, das an Mäusen stark cancerogene Wirkung zeigt (C.A. 77, 97 540 b).

Die Verwendung von trisubstituierten Phosphinen oder Phosphinoxiden zur Herstellung von Hydroxybenzolsulfonsäurehalogeniden aus den zugrundeliegenden Sulfonsäuresalzen und Thionylchlorid, Phosphoroxychlorid oder Phosgen ist aus US 3 673 247 bekannt. Weiterhin ist der Einsatz von Phosphinchloridverbindungen und Phosphinoxidverbindungen für die Herstellung von Carbonsäurechloriden aus den Carbonsäuren bekannt (DE-OS 2 841 069, DE-OS 2 321 122, US 3 544 626, US 4 129 595).

Die Reaktion von Phenolen mit Phosgen in Gegenwart von Natronlauge und in Gegenwart quartärer Phosphorverbindungen führt zur Bildung von Diarylcarbonaten (DE-OS 2 804 227, französisches Patent 1 381 791).

Weiterhin ist bekannt, dass aliphatische Alkohole mit Phosgen zur Chloroformaten reagieren, wenn Trialkylphosphit oder $PCl_3$, das unter den Reaktionsbedingungen Trialykylphosphit bildet, zugegeben wird (japanische Patentanmeldungen 23 409/67 und 7890/67). Aliphatische Alkohole lassen sich jedoch auch ohne Zusätze zu Chloroformaten umsetzen, so dass ein technischer Fortschritt durch die Zusätze von Phosphit oder $PCl_3$ nicht erkennbar ist. Phenol reagiert dagegen bei den in den japanischen Patentanmeldungen angegebenen Temperaturbereichen bis zu 35°C in Anwesenheit von Triethylphosphit nicht mit Phosgen, während sich bei erhöhter Temperatur das Diphenylcarbonat bildet.

Es wurde nun ein Verfahren zur Herstellung aromatischer Chlorameisensäurearylester aus Phenolen und Phosgen gefunden, das dadurch gekennzeichnet ist, dass die Umsetzung in homogener flüssiger Phase bei einer Temperatur von 60 bis 180°C in Gegenwart organischer Phosphorverbindungen der Formel

$$R^1R^2R^3PR^4{}_nX_n \qquad (I),$$

in der

$R^1$, $R^2$ und $R^3$ unabhängig voneinander für Wasserstoff, Alkyl, Alkenyl, Aralkyl, Aryl oder homöopolar an Phosphor gebundenes Halogen stehen und wobei zwei der genannten Reste mit dem Phosphoratom einen 5- oder 6-gliedrigen phosphorhaltigen gesättigten oder ungesättigten Heterocyclus bilden können,

X für OH, homöopolar gebundenes Halogen oder ein anorganisches oder organisches Säureanion steht,

$R^4$ Wasserstoff oder Alkyl bedeutet oder, wenn X

Halogen bedeutet, auch Halogen bedeuten kann
n 0 oder 1 bedeutet,
und in der weiterhin $R^4$ und X gemeinsam für Sauerstoff oder Schwefel stehen können, wobei die organische Phosphorverbindung jedoch maximal zwei Halogenatome enthält, durchgeführt wird.

Als Alkyl sei beispielsweise ein geradkettiger, verzweigter oder cyclischer Rest mit bis zu 12, bevorzugt 8, besonders bevorzugt 6, Kohlenstoffatomen genannt, wie Methyl, Ethyl, Propyl, Isopropyl, Butyl, Isobutyl, tert.-Butyl, Pentyl, Hexyl, Decyl, Dodecyl, Cyclohexyl, 4-Ethyl-cyclohexyl. Bevorzugte Alkylreste sind Butyl oder Cyclohexyl.

Als Alkenyl seien beispielsweise geradkettige, verzweigte oder cyclische Reste mit wenigstens einer olefinischen Doppelbindung und 2 bis 12, bevorzugt bis 8, besonders bevorzugt bis 6, Kohlenstoffatomen genannt, wie 1-Propenyl, 2-Propenyl, Butenyl, 3-Methyl-2-butenyl, Hexenyl. Bevorzugtes Alkenyl ist Propenyl oder Butenyl.

Als Aralkyl seien Kohlenwasserstoffreste mit einem aliphatischen und einem aromatischen Teil genannt, wie Benzyl, Phenylethyl, Naphthylmethyl, Naphthyl-ethyl, 9-Fluorenyl, bevorzugt Benzyl.

Als Aryl seien aromatische Kohlenwasserstoffreste mit bis zu 15, bevorzugt bis zu 10, besonders bevorzugt bis zu 7 Kohlenstoffatomen genannt, wie Phenyl, Tolyl, Naphthyl, Ethylnaphthyl, Anthryl, Methylanthryl, Fluorenyl oder Biphenyl, bevorzugt Phenyl oder Tolyl.

Als Halogen sei beispielsweise Chlor oder Brom, bevorzugt Chlor, genannt. Die organische Phosphorverbindung enthält jedoch maximal zwei Halogenatome.

Für den Fall, dass zwei der Reste $R^1$ bis $R^3$ mit dem Phosphor einen 5- oder 6-gliedrigen phosphorhaltigen gesättigten oder ungesättigten Heterocyclus bilden, seien beispielsweise das Phosphol-System, das Phospholen-System, das Phospholan-System, das Dibenzophospholan-System oder das Phosphacyclohexan-System, bevorzugt das Phospholen- oder das Phospholan-System, genannt.

Der Substituent X in der Forml (I) kann neben Hydroxyl auch homöopolar gebundenes Halogen, beispielsweise Chlor oder Brom, bedeuten. Der Substituent X kann auch das organische oder organische Säureanion eines Phosphoniumsalzes sein, beispielsweise Chlorid, Bromid, Sulfat, Phosphat, Methylsulfat oder aliphatisches oder aromatisches Sulfonat.

Weiterhin können die Reste $R^4$ und X gemeinsam für Sauerstoff oder Schwefel, bevorzugt für Sauerstoff, stehen.

n bedeutet 0 oder die Zahl 1, und zwar jeweils gemeinsam für den Rest $R^4$ und X.

Unter die organischen Phosphorverbindungen der Formel (I) fallen beispielsweise Phosphine, Phosphinoxide, Phosphinsulfide, Phosphinhalogenide, Phosphoniumsalze oder Halogenphosphine.

Als Phosphine seien beispielsweise genannt: Tributylphosphin, Triphenylphosphin, Methyldiphenylphosphin, Diethylcyclohexylphosphin, Tricyclohexylphosphin, Allylbutyl-phenylphosphin, Methyl-benzyl-tolylphosphin, 1-Methylphosphol, 1-Methyl-2,5-dihydrophosphol, 1-Phenyl-2-methyl-2,5-dihydrophosphol, 1-Methyl-dibenzophospholan. Bevorzugte Phosphine sind Tributylphosphin, Triphenylphosphin, 1-Phenyl-2-methyl-2,5-dihydro-phosphol.

Als Phospinoxide seien beispielsweise genannt:

1-Methylphospholoxid, 1-Phenyl-2-methyl-2,5-dihydrophospholoxid, Tributylphosphinoxid, Triphenylphosphinoxid, Triphenylphosphinoxid-hydrat, bevorzugt Triphenylphosphinoxid.

Als Phosphinsulfid sei beispielsweise das Tributylphosphinsulfid genannt.

Als Phosphinhalogenide seien beispielsweise Triphenylphosphindichlorid, Triphenylphosphindibromid, Tributylphosphindichlorid und Tributylphosphindibromid, bevorzugt Triphenylphosphindichlorid und Tributylphosphindichlorid, genannt.

Als Phosphoniumsalze seien beispielsweise Methyltriphenylphosphoniumbromid und-chlorid, Tributylphosphoniumchlorid, Tetraethylphosphoniumchlorid, Methyltrioctylphosphoniumjodid, Allyltriphenylphosphoniumchlorid und Methyltributylphosphoniummethosulfat, bevorzugt Methyltriphenylphosphoniumbromid und -chlorid, genannt.

Als Halogenphosphine seien beispielsweise Diphenylchlorphosphin und Phenyldichlorphosphin, bevorzugt Phenyldichlorphosphin, genannt.

Unter den genannten Verbindungsklassen der organischen Phosphorverbindungen sind Alkyl- und Arylphosphine, Alkyl- und Arylphosphinoxide sowie die Phosphinhalogenide bevorzugt, die Phosphine und Phosphinoxide besonders bevorzugt.

Als organische Phosphorverbindungen werden daher im erfindungsgemässen Verfahren bevorzugt solche der Formel

$$R^5R^6R^7PR^8{}_nY_n \qquad (II)$$

eingesetzt, in der
$R^5$, $R^6$ und $R^7$ unabhängig voneinander für Alkyl, Benzyl, Phenyl oder homöopolar an Phosphor gebundenes Chlor stehen,
Y für OH, Chlor oder Brom steht,
$R^8$ Wasserstoff oder Alkyl bedeutet, oder wenn Y Chlor oder Brom ist, auch Chlor oder Brom bedeuten kann,
n die obengenannte Bedeutung hat und
in der weiterhin $R^8$ und Y gemeinsam für Sauerstoff stehen können, wobei die organische Phosphorverbindung jedoch maximal zwei Chlor- bzw. Bromatome enthält.

Besonders bevorzugt werden im erfindungsgemässen Verfahren organische Phosphorverbindungen der Formel

$$R^9R^{10}R^{11}PR^{12}{}_nZ_n \qquad (III),$$

eingesetzt, in der

$R^9$, $R^{10}$ und $R^{11}$ unabhängig voneinander Alkyl oder Phenyl bedeuten,
$R^{12}$ Chlor bedeutet,
Z Chlor bedeutet,
n die obengenannte Bedeutung hat und
in der weiterhin $R^{12}$ und Z gemeinsam für Sauerstoff stehen können.

Selbstverständlich können auch Gemische der genannten organischen Phosphorverbindungen eingesetzt werden.

Weiterhin ist es möglich, Verbindungen einzusetzen, aus denen unter den Reaktionsbedingungen die angegebenen organischen Phosphorverbindungen entstehen können. Für den letzteren Fall sei beispielsweise die Umsetzung von trisubstituierten Phosphinen mit dem bei der Reaktion entstehenden Chlorwasserstoff zum entsprechenden Phosphoniumsalz und auf die Reaktion von Phosphinoxiden mit Phosgen zu Phosphinhalogeniden und Kohlendioxid verwiesen.

Als Phenole für das erfindungsgemässe Verfahren können beispielsweise solche der Formel

$$R^{13}R^{14}R^{15}AR^1(OH)_m \quad (IV),$$

eingesetzt werden, in der
$R^{13}$, $R^{14}$ und $R^{15}$ unabhängig voneinander für Wasserstoff, Alkyl, Halogenalkyl, Aralkyl, Aryl, Halogen, Nitro, Cyano, Alkoxy, Alkylthio, Aralkoxy, Aralkylthio, Aryloxy, Arylthio, Alkylthioalkyl, Alkoxyalkyl, Halogenalkylalkoxy, Trimethylsilyl, Carboxyl, Carboalkoxy, gegebenenfalls derivatisiertes Formyl, gegebenenfalls derivatisiertes Alkylamino, Alkyl-alkinyl-amino oder Dialkylamino stehen,
$Ar^1$ den Benzolkern, den Naphthalinkern, den Anthracenkern, das Bisphenylalkylidengerüst, das Diphenyloxidgerüst, das Diphenylsulfidgerüst, das Diphenylsulfongerüst, das Diphenylamingerüst, einen heterocyclischen aromatischen 5- oder 6-Ring oder ein benzokondensiertes heterocyclisches Gerüst bedeutet,
m eine Zahl von 1 bis 3 darstellt
und wobei die OH-Gruppen bis auf eine derivatisiert sein können.

Bevorzugt werden Phenole der Formel

$$R^{16}R^{17}Ar^2(OH)_p \quad (V)$$

eingesetzt, in der
$R^{16}$ oder $R^{17}$ für Wasserstoff, Alkyl, Halogenalkyl, Halogen, Nitro, Cyano oder Alkoxy steht,
$Ar^2$ den Benzolkern, den Naphthalinkern, das Chinolingerüst, das Benzothiophengerüst, das Benzodihydrofurangerüst, das Benzodihydropyrangerüst, das Bisphenyl-alkylidengerüst oder das Diphenyloxidgerüst bedeutet und
p die Zahl 1 oder 2 darstellt.

Für das erfindungsgemässe Verfahren werden in besonders bevorzugter Weise Phenole der Formel

$$R^{18}\text{-}C_6H_4OH \quad (VI)$$

eingesetzt, in der
$R^{18}$ Wasserstoff, $C_1$–$C_4$-Alkyl, $CF_3$, $CCl_3$, Cl, Br, $NO_2$ oder $C_1$–$C_4$-Alkoxy bedeutet.

Als Bedeutungsumfang für Alkyl, Aralkyl, Aryl sei beispielsweise auf den bereits genannten Umfang verwiesen.

Als Halogenalkyl seien beispielsweise fluorierte, chlorierte oder bromierte Alkylreste mit bis zu 12, bevorzugt bis zu 6, bevorzugt bis zu 4, Kohlenstoffatomen genannt, wie Trifluormethyl, Trichlormethyl, Tribrommethyl, die verschieden hoch fluorierten, chlorierten oder bromierten Ethyl-, Propyl-, Butyl-, Hexyl-, Decyl- oder Dodecylreste.

Als Halogensubstituenten für die erfindungsgemäss einsetzbaren Phenole sei ausser Chlor und Brom noch Fluor und Jod, bevorzugt Fluor, Chlor oder Brom, besonders bevorzugt Chlor oder Brom, genannt.

Als Alkoxy seien beispielsweise Reste aliphatischer Alkohole mit bis zu 12, bevorzugt bis zu 6, bevorzugt bis zu 4, Kohlenstoffatomen genannt, wie Methoxy, Ethoxy, Propoxy, Butoxy, Hexyloxy, Decyloxy oder Dodecyloxy.

Als Alkylthio seien beispielsweise die Thioanaloga zu den genannten Alkoxyresten genannt.

Als Aralkoxy seien Reste araliphatischer Alkohole genannt, wie Benzyloxy, Phenyl-ethoxy, Naphthyl-methoxy, Naphthyl-ethoxy, bevorzugt Benzyloxy.

Als Aralkylthio seien beispielsweise die Thioanaloga der genannten Aralkoxyreste genannt.

Als Aryloxy seien beispielsweise Reste von Phenolen genannt, wie Phenoxy, Naphthyloxy, Anthryloxy, bevorzugt Phenyloxy.

Als Arylthio seien beispielsweise die Thioanaloga der genannten Aryloxyreste genannt.

Als Carboalkoxy seien beispielsweise Estergruppen mit bis zu 4 Kohlenstoffatomen genannt, wie Carbomethoxy, Carboethoxy oder Carbopropoxy.

Für den Fall, dass die Formylgruppe derivatisiert ist, sei beispielsweise die 1,3-Dioxolan-2-yl- oder 4,5-Dimethyl-1,3-dioxolan-2-yl-Gruppe genannt.

Als Dialkylamino sei beispielsweise Dimethylamino, Diethylamino, Dipropylamino, Dibutylamino, Methylethylamino, Methylpropylamino oder Methylbutylamino, genannt. In Alkyl-alkinyl-amino-Gruppen seien neben den genannten Alkylresten beispielsweise Äthinyl, Propinyl oder Butinyl genannt.

Als Alkylamino sei mit nur einem der genannten Alkylreste substituiertes Amino genannt. Für den Fall, dass eine Amino- oder Alkylamino-Gruppe derivatisiert ist, sei beispielsweise ein Amid, wie Acetamid, ein Imin oder Amidin oder deren Salz mit Halogenwasserstoff oder Schwefelsäure oder die Carbamatgruppe, wie Methylcarbamat oder Äthylcarbamat, genannt.

Für den Fall, dass $Ar^1$ das Bisphenylalkylidengerüst darstellt, sei für die Alkylidengruppe ein Rest mit bis zu 8 Kohlenstoffatomen genannt, wie die Methylengruppe, die 1,1-Ethylidengruppe, die 1,2-Ethylengruppe, die 2,2-Propylidengruppe, die 1,1,3-Trimethyltrimethylengruppe oder die Cyclohexylidengruppe, bevorzugt die Methylengruppe, die 1,1-Ethylidengruppe oder die 2,2-Propylidengruppe. Als heterocyclischer aromatischer 5- oder 6-Ring oder als benzokondensiertes heterocyclisches Gerüst sei beispielsweise Pyrrol, Furan, Thiophen, Pyridin, Chinolin, Isochinolin, Indol, Cumaron, Thionaphthen, Acridin, Benzopyran genannt.

m bedeutet eine der Zahlen 1,2 oder 3;

p bedeutet 1 oder 2.

Für den Fall, dass $Ar^1$ ein mehrkerniges aromatisches Gerüst darstellt, können die Reste $R^{13}$ bis $R^{17}$ und die bis zu 3 Hydroxylgruppen gleichmässig oder unterschiedlich auf die verschiedenen Kerne eines solchen Gerüstes in den Formeln (IV) und (V) verteilt sein.

Die OH-Gruppen können bis auf eine auch derivatisiert sein, beispielsweise durch Veresterung, so mit Essigsäure oder Phosphorsäure oder als gemischtes Alkylacetal, beispielsweise des Chloracetaldehydes vorliegen oder, im Falle zweier benachbarter OH-Gruppen, Teile eines Dioxolanringes sein.

Beispiele für einzelne erfindungsgemäss einsetzbare Phenole sind: Phenol, o-Kresol, m-Kresol, p-Kresol, Xylenole, 4-Chlorphenol, Hydrochinon, Resorcin, 1-Naphthol, 2-Naphthol, 1,5-Dihydroxynaphthalin, 4,4'-Methylenbisphenol, 1,1-Bis-(4-hydroxyphenyl)-ethan, 2,2-Bis-(4-hydroxyphenyl)-propan, 4,4'-Cyclohexyliden-bisphenol, 4,4'-Dihydroxydiphenylether, 4,4'-Dihydroxydiphenylsulfid, 4,4'-Dihydroxydiphenylsulfon, 4,4'-Dihydroxydiphenyl-N-methyl-amin, 2-sec.-Butylphenol, 3-sec.-Butylphenol, 3,5-Di-i-propylphenol, 3,4-Dimethylphenol, 3,5-Dimethylphenol, 3,5-Di-tert.-butylphenol, 3-Methylphenol, 3-(2-Pentyl)-phenol, 3-(3-Pentyl)-phenol, 3,4,5-Trimethylphenol, 2-i-Propylphenol, 3-i-Propylphenol, 3-tert.-Butylphenol, 3-Methyl-5-i-propyl-phenol, 1-Naphthol, 5,6,7,8-Tetrahydro-1-naphthol, 3,4-Dimethyl-6-chlorphenol, 2-Chlorphenol, 3,5-Dimethyl-4-dimethylamino-phenol, 3,5-Dimethyl-4-diallylamino-phenol, 3,5-Dimethyl-4-methylthiophenol, 2-Methyl-4-dimethylamino-phenol, 2-Isopropoxy-phenol, 3-Aminophenol, Amino-phenole, bei denen die $NH_2$-Gruppe als Acetat oder als Imin- oder Amidinsalz oder als Methylcarbamat geschützt ist, 2-(1',3'-Dioxolan-2'-yl)-phenol, 2-(4',5'-Dimethyl-1',3'-dioxolan-2'-yl)-phenol, 3-Methyl-4-dimethylaminophenol, 2-Thiobutyl-phenol, 2-Thioethyl-methylphenol, 3,5-Dimethyl-4-(N,N-dimethylamino)-phenol, 3,5-Dimethyl-4-amino-phenol, 2-tert.-Butyl-5-methyl-dimethylamino-phenol, 2-(N-Methyl-N-propinyl)-amino-phenol, 2-Allyloxyphenol, 2-Hydroxy-phenol, 2,3-Hydroxy-phenol, 3-Trimethylsilyl-phenol, Polyhydroxyphenole, bei denen die OH-Gruppen bis auf eine beispielsweise als Acetal des Acetons, als gemischtes Acetal des Chloracetalaldehyds oder als gemischter Äthylester der Phosphorsäure geschützt sind, sowie 1-Chinolin-8-ol, 2-Methyl-1-chinolin-8-ol, Benzothiophen-4-ol, 2,2-Dimethyl-7-benzofuranol, 4-Chlor-2,3-dihydro-2,2-dimethyl-7-benzofuranol, 3,4-Dihydro-2,2-dimethyl-benzopyran-8-ol.

Das erfindungsgemässe Verfahren sei anhand der folgenden Formelgleichung am Beispiel der Umsetzung von Phenol mit Phosgen in Gegenwart einer organischen Phosphorverbindung erläutert:

$$C_6H_5OH + COCl_2 \xrightarrow[\text{P-Verbindung}]{\text{organische}} C_6H_5O\text{-}COCl + HCl$$

Die erfindungsgemäss einzusetzende organische Phosphorverbindung wird in einer Menge von beispielsweise 0,1 bis 20 Mol-%, bevorzugt 0,2 bis 10 Mol-%, besonders bevorzugt 0,5 bis 5 Mol-% pro Äquivalent organische Hydroxylgruppe, eingesetzt.

Unter der Einwirkung der erfindungsgemässen Zusätze einer organischen Phosphorverbindung wird Phosgen im Molverhältnis von 1:1 bis 2:1, bevorzugt 1:1 bis 1,5:1, besonders bevorzugt 1:1 bis 1,2:1, bezogen auf jedes eingesetzte Phenoläquivalent, verwendet. Die Verwendung von weniger als der stöchiometrischen Menge Phosgen ist möglich, bringt aber im allgemeinen keinen Vorteil, da hierbei nicht umgesetztes Phenol abgetrennt werden muss und die Ausbeute sinkt. Die Verwendung grösserer Mengen an Phosgen ist möglich, bringt aber keine weiteren Vorteile.

Das erfindungsgemässe Verfahren wird in homogener flüssiger Phase durchgeführt. Als homogene flüssige Phase kann beispielsweise die Schmelze der beschriebenen Phenole oder eine Lösung der beschriebenen Phenole in einem inerten Lösungsmittel angesehen werden. Für den Fall, dass in Lösung gearbeitet wird, kann als inertes Lösungsmittel beispielsweise ein aliphatischer oder aromatischer Kohlenwasserstoff, wie Pentan, Hexan, Cyclohexan, Toluol, Xylol oder Benzol, ein halogenierter Kohlenwasserstoff, wie Trichlorethan, Chlorbenzol oder Dichlorbenzol oder ein Ester, wie Ethylacetat oder Butylacetat, eingesetzt werden. In bevorzugter Weise wird ein halogenierter Kohlenwasserstoff, besonders bevorzugt Chlorbenzol, eingesetzt.

In bevorzugter Weise wird ohne Lösungsmittel nur in der Schmelze des Phenols gearbeitet. Hierdurch wird eine höhere Raumausbeute erzielt als beim Arbeiten in Gegenwart eines Lösungsmittels. Jedoch kann das Arbeiten im Lösungsmittel dann vorteilhaft sein, wenn der Schmelzpunkt des einzusetzenden Phenols oberhalb der angestrebten Reaktionstemperatur liegt und das Phenol daher, zumindest zu Beginn der Reaktion, ohne Gegenwart des Lösungsmittels nur langsam mit dem Phosgen reagieren würde. Die Gegenwart eines Lösungsmittels kann auch zur Beherrschung und Abführung der Reaktionswärme der exothermen Reaktion günstig sein.

Die Reaktion des erfindungsgemässen Verfahrens wird im Temperaturbereich von 60 bis 180°C, bevorzugt von 80 bis 160°C, besonders bevorzugt von 100 bis 140°C, durchgeführt.

Die Reaktion kann unter Normaldruck oder unter erhöhtem Druck durchgeführt werden. Die Erhöhung des Partialdruckes an Phosgen in der Reaktionsmischung bei erhöhtem Druck innerhalb einer geschlossenen Apparatur erhöht die Reaktionsgeschwindigkeit. Durch die Bildung von Chlorwasserstoff im Laufe des Fortschreitens der Reaktion steigt allerdings der Gesamtdruck in einer geschlossenen Apparatur stark an, was zusätzlichen apparativen und sicherheitstechnischen Aufwand erfordert. Im allgemeinen wird man daher die Reaktion unter atmosphärischem Druck oder unter nur leicht erhöhtem Druck, beispielsweise bis 10 bar, durchführen, wobei der Chlorwasserstoff diskontinuierlich oder kontinuierlich aus der Druckapparatur ausgeschleust werden kann.

Das erfindungsgemässe Verfahren kann kontinuierlich oder diskontinuierlich durchgeführt werden. Ein kontinuierliches Verfahren kann beispielsweise im Reaktionsrohr, in einer Rührkesselkaskade, in einem Schlaufenreaktor oder in einer Gegenstromkolonne durchgeführt werden.

Zur Durchführung des erfindungsgemässen Verfahrens kann beispielsweise das Phenol und die vorgesehene organische Phosphorverbindung vorgelegt werden und unter Aufschmelzen auf die gewünschte Reaktionstemperatur gebracht werden. Bei dieser Temperatur wird Phosgen eingeleitet, so dass an dem aufgesetzten Rückflusskühler nur ein schwacher Rückfluss von Phosgen auftritt. An einem Blasenzähler oder einer anderen geeigneten Vorrichtung wird sodann die Entwicklung von Chlorwasserstoff beobachtet. Die Reaktion ist dann beendet, wenn die Chlorwasserstoffentwicklung aufhört. Das Reaktionsgemisch wird sodann durch Destillation in den gewünschten Chlorameisensäureester und einen Rückstand aufgetrennt. Dieser Rückstand enthält die organische Phosphorverbindung. Die organische Phosphorverbindung hat hierdurch ihre Wirksamkeit für das erfindungsgemässe Verfahren nicht verloren. Sie kann daher für einen weiteren Lauf des erfindungsgemässen Verfahrens eingesetzt werden. Hierzu kann der Sumpf aus der Destillation des ersten Reaktionslaufes, beispielsweise durch weitere Destillation oder durch geeignete Umkristallisation, auf die reine organische Phosphorverbindung aufgearbeitet werden, die sodann für einen weiteren Reaktionslauf zur Verfügung steht. In einer weiteren Variante des erfindungsgemässen Verfahrens ist es jedoch auch möglich, den Destillationssumpf aus dem ersten Reaktionslauf, der die noch immer wirksame organische Phosphorverbindung enthält, unverändert in einem zweiten Reaktionslauf zu benutzen. Wegen der Einfachheit ist diese letztere Variante bevorzugt. Es ist jedoch auch möglich, gegebenenfalls gebildete und im Rückstand verbleibende Nebenprodukte teilweise auszuschleusen und nur einen Teil des Rückstandes mit darin enthaltener organischer Phosphorverbindung in einen Folgeansatz zuurückzuführen. Die wiederholte Einsetzbarkeit der organischen Phosphorverbindung kann beispielsweise 2 bis 15mal, bevorzugt 2 bis 10mal, besonders bevorzugt 3 bis 6mal, durchgeführt werden. Eine solche wiederholte Benutzung der organischen Phosphorverbindung ist besonders bedeutungsvoll, wenn das erfindungsgemässe Verfahren kontinuierlich durchgeführt wird. Hierbei kann dann beispielsweise das aus dem kontinuierlich betriebenen Reaktor entnommene Reaktionsgemisch, in dem die Reaktion nicht notwendigerweise bis zum vollständigen Umsatz durchgeführt wurde, in eine kontinuierlich betriebene Destillationseinrichtung eingespeist werden. Bei dieser kontinuierlichen Destillation wird als Kopfprodukt der gewünschte Chlorameisensäurearylester entnommen, während das Sumpfprodukt, zumindest teilweise, in das Ausgangsgemisch für das erfindungsgemässe Verfahren zurückgeführt wird.

Als Chlorameisensäurearylester, die nach dem erfindungsgemässen Verfahren hergestellt werden können, seien beispielsweise genannt: Chlorameisensäurephenylester, -o-kresylester, -m-kresylester, p-kresylester, -dimethylphenylester, -4-chlorphenylester, -naphthylester, Hydrochinon-, Resorcin-, 1,5-Dihydroxynaphthalin-bis-chloroformat, 4,4'-Methylbisphenyl-, 1,1-Bis-(4-hydroxyphenyl)-ethan-, 2,2-Bis-(4-hydroxyphenyl)-propan-, 4,4'-Cyclohexylidenbisphenyl-, 4,4'-Dihydroxdiphenylether-bis-chloroformat.

Die Chlorameisensäurearylester, insbesondere der Chlorameisensäurephenylester, werden nach dem erfindungsgemässen Verfahren in hoher Reinheit erhalten. Sie können daher für viele Zwecke als Rohprodukte weiterverwendet werden. Selbstverständlich können sie in bekannter Weise, beispielsweise durch Destillation oder Umkristallisation, auch weiter gereinigt werden.

Chlorameisensäurearylester sind wertvolle Zwischenprodukte, beispielswesie bei der Herstellung von Farbstoffen, wie Sirius-Farbstoffe (DE-OS 2325088, Ullmanns Enzykopädie der technischen Chemie, Band 4, Seite 105, 108 und 109, Urban und Schwarzenberg, 3. Auflage, Berlin-München, 1953), zur Herstellung von Polycarbonatkunststoffen, Bakteriziden und Pflanzenschutzmitteln, wie Herbiziden und Insektiziden, besonders aus der Klasse der Carbamate, wie sie z.B. in «The Pesticide Manual» (British Crop Protection Council), 6. Auflage, 1979, oder in K.H. Büchel, «Pflanzenschutz und Schädlingsbekämpfung» (G. Thieme Verlag, 1977, S. 60–72) aufgeführt sind.

Besonders seien die Verbindungen genannt, die unter folgenden Handelsnamen bzw. «common-names» aufgeführt sind: Sevin (Carbaryl), Mexacarbate, Methiocarb, Baycarb, Aminocarb (Matacil), Propoxur (Baygon), Carbanolate, Promecarb, Mobam, Allyxycarb (Hydrol), Butacarb, Carbofuran, Formetamatehydrochlorid, Meobal, Metacrate, Bufencarb, Dioxacarb, Macbal, Landrin, CMPO, Isoprocarb, Sapecron, TBPMC, Bendiocarb, Ethiofencarb, Hercules 6007, Promacyl.

Solche Carbamate können auch durch Umsetzung von Phenolen mit Carbamoylhalogeniden oder Isocyanaten erhalten werden. Carbamoylhalogenide sind jedoch arbeitshygienisch bedenkliche Verbindungen (s. S. 3). Mit Isocyanaten erhält man zunächst nur solche Carbamate, die am Carbamat-N-Atom ein H-Atom tragen; beim Umsatz von Chloroformaten mit Aminen unterliegt man dieser Einschränkung nicht, so dass dieser Syntheseweg besondere Vorteile mit sich trägt, ganz besonders zur Herstellung von am Carbamat-N-Atom disubstituierter Carbamate. Die genannten Verwendungszwecke sind dem Fachmann bekannt und beispielsweise beschrieben in DE-AS 2 131 555, DE-AS 1 213 419 und Ullmanns Enzyklopädie der technischen Chemie, 4. Auflage, Band 9, Seite 383, Verlag Chemie 1975.

Gegenüber dem Stand der Technik bietet das erfindungsgemässe Verfahren folgende Vorteile: Man erhält höhere Ausbeuten und reinere Produkte; die Korrosion ist geringer, ebenso die ökologischen und arbeitshygienischen Probleme; es kann auch ohne Anwendung von Druck gearbeitet werden, was eine höhere Sicherheit der Verfahrensführung mit sich bringt; für den Fall, dass ohne zusätzliche Lösungsmittel gearbeitet wird, wird eine höhere Raum/Zeit-Ausbeute erzielt; die Möglichkeit der wiederholten Einstäze der erfindungsgemässen organischen Phosphorverbindungen senkt die Kosten des Verfahrens.

Es ist überraschend, dass die erfindungsgemässen organischen Phosphorverbindungen die Reaktion des Phenols mit dem Phosgen zum Chlorameisensäurearylester bewirken, jedoch die weitere Kondensation zum Diarylcarbonat nicht fördern. Überraschenderweise sind die erfindungsgemässen Zusätze den bekannten Zusätzen nach dem Stand der Technik in ihrer Auswirkung auf die Reaktion überlegen, zumindest jedoch gleichwertig, zeigen jedoch zusätzlich die aufgezeigten technischen Vorteile.

Vergleichsbeispiele

Beispiel 1

(Durchführung entsprechend DAS 2 131 555, Beispiel 1):

In einem Rundkolben, der mit einem Rührer, Gaseinleitungsrohr, Innenthermometer und einem auf −30 bis −40°C gekühlten Rückflusskühler mit Blasenzähler in der Gasableitung versehen ist, wird ein Gemisch aus 500 g Phenol (5,32 Mol) und 10 bis 20 g Dimethylformamid auf 100 bis 120°C erhitzt. Man leitet langsam Phosgen ein, so dass am Rückflusskühler gerade kein Rückfluss beobachtet wird. Dabei verfärbt sich der Kolbeninhalt dunkel.

Die Reaktion ist weitgehend beendet, wenn dauerhaft Rückfluss von $COCl_2$ beobachtet wird. Man rührt dann noch ½ bis 1 Stunde nach oder entfernt den Rückflusskühler und leitet noch weiteres Phosgen durch (10 bis 20% Überschuss).

Man bläst überschüssiges Phosgen mit Stickstoff aus dem Reaktonsgemisch aus und destilliert Chlorameisensäurephenylester im Vakuum ab (Kp. 85°C/26,6 mbar [= 2,66 kPa)].

Man erhält 761,5 g eines (laut Titration mit Di-n-butylamin) 99,5%igen Produktes (91% der theoretischen Ausbeute).

Es enthält laut gaschromatographischer Analyse ca. 0,1 bis 0,2% Dimethylcarbamoylchlorid.

Beispiel 2

(Durchführung entsprechend japanischer Patentanmeldung 23 409/67):

In einer Apparatur wie in Beispiel 1 legt man 94 g (1 Mol) Phenol und 8,3 g Triethylphosphit vor, verflüssigt durch Aufschmelzen und lässt auf 35°C abkühlen. Dabei erstarrt die Mischung wieder. Man leitet Phosgen ein, bis Rückfluss eintritt. Am Blasenzähler wird keine Entwicklung von HCl beobachtet; es tritt also keine wesentliche Reaktion ein.

Beispiel 3

Man geht vor, wie in Beispiel 2 beschrieben, erhitzt jedoch auf 120 bis 125°C und leitet innerhalb von 8 Stunden ca. 60 g $COCl_2$ ein.

Man bläst überschüssiges $COCl_2$ aus und erhält 104 g Rohprodukt, das beim Stehen erstarrt.

Es enthält ca. 15% Chlorameisensäurephenylester [das entspricht einer Ausbeute von 10% der theoretischen Ausbeute und besteht im wesentlichen aus Diphenylcarbonat (laut gaschromatographischer Analyse).

Erfindungsgemässe Beispiele

Beispiel 4

a) In einer Apparatur, wie in Beispiel 1 beschrieben, legt man 500 g (5,32 Mol) Phenol und 28 g Triphenylphosphin vor, erwärmt auf 120 bis 125°C und leitet bei dieser Temperatur Phosgen ein. Innerhalb von 8 bis 10 Stunden werden 530 g $COCl_2$ verbraucht. Man rührt noch 1 Stunde bei 125°C nach, ersetzt dann den Rückflusskühler durch ein Gaseinleitungsrohr und bläst überschüssiges $COCl_2$ mit Stickstoff aus.

Im Gegensatz zur Durchführung nach Beispiel 1 und 3 ist der Reaktionsansatz auch jetzt noch farblos bis hellgelb.

Man destilliert im Vakuum bei 74 bis 79°C/16 mbar (= 1,6 kPa) bis zu einer Innentemperatur von 105°C und erhält 744 g Chlorameisensäurephenylester (Gehalt 99,6% laut Amintitration; Ausbeute 89,5% der theoretischen Ausbeute).

b) Der Rückstand von 100 g wird ohne Neueinsatz von Triphenylphosphin mit weiteren 500 g Phenol versetzt. Man führt die Reaktion, wie unter 4a) beschrieben, mit 560 g $CoCl_2$ aus. Nach einer Phosgenierzeit von 8 Stunden erhält man 764 g destillierten 99,7%igen Chlorameisensäurephenylester (91,5% der theoretischen Ausbeute).

c) Der Destillationsrückstand wird ohne Neueinsatz von Triphenylphosphin erneut mit 500 g Phenol und 540 g Phosgen eingesetzt. Man erhält nach einer Phosgenierzeit von 10 Stunden 754 g destillierten Chlorameisensäurephenylester (Gehalt 99,8%; Ausbeute 90,4% der theoretischen Ausbeute).

Es hinterbleibt ein dünnflüssiger Destillationsrückstand von ca. 200 g, der noch 25% Chlorameisensäurephenylester enthält; der Rest ist im wesentlichen Diphenylcabonat. Die Ausbeute nach 3 Zyclen beträgt also 2262 g, Reinheit etwa

99,7%ig (90,3% der theoretischen Ausbeute) im Destillat, zuzüglich ca. 50 g im Rückstand (2,0% der theoretischen Ausbeute).

Beispiel 5

Man geht vor, wie in Beispiel 4a) beschrieben, verwendet jedoch nur 7,0 g Triphenylphosphin und leitet 550 g $COCl_2$ bei ca. 145°C ein.

Nach ca. 14stündiger Reaktion erhält man durch Destillation 640,6 g 99,6%igen Chlorameisensäurephenylester und 147,2 g Rückstand, der noch 28,4% Ester enthält. Die Gesamtausbeute beträgt also 638,0 g (76,6% der theoretischen Ausbeute), zuzüglich 41,8 g im Rückstand (5,0% der theoretischen Ausbeute).

Beispiel 6

In einen Ansatz, wie in Beispiel 4a) beschrieben, leitet man, zunächst ohne zu erwärmen, Phosgen ein, bis genügend $COCl_2$ in der Apparatur anwesend ist, um unter Rückfluss zu sieden. Man unterbricht die Gaseinleitung und erwärmt langsam.

Ab 60°C Innentemperatur kann man am Kühlerausgang entweichendes HCl-Gas nachweisen; das bedeutet, dass die Reaktion bereits bei dieser Temperatur abläuft.

Ab 80°C Innentemperatur hört auch der Rücklauf von $COCl_2$ am Kühler auf, da die Reaktion schnell genug abläuft, um den vorhandenen Vorrat an $COCl_2$ innerhalb einiger Minuten zu verbrauchen.

Beispiel 7

a) Man arbeitet wie im Beispiel 4a) beschrieben, verwendet jedoch 30 g technisches Triphenylphosphinoxid anstatt von Triphenylphosphin.

Während einer Umsetzungszeit von 12 Stunden werden 530 g $COCl_2$ aufgenommen.

Durch Destillieren erhält man 718 g Chlorameisensäurephenylester (Gehalt 99,5%; Ausbeute 85,7% der theoretischen Ausbeute).

b) Der Rückstand von 110 g wird ohne Zusatz weiteren Triphenylphosphinoxids in einen neuen Ansatz von 500 g Phenol eingebracht. In 12 Stunden werden 550 g $COCl_2$ aufgenommen. Man entfernt den Kühler und leitet weitere 100 g $COCl_2$ durch den Ansatz. Durch Destillation erhält man 733,1 g Chlorameisensäurephenylester (Gehalt 99,2%; Ausbeute 87,4% der theoretischen Ausbeute). Es hinterbleibt ein in der Wärme dünnflüssiger Rückstand von 170 g, der noch 25% Chlorameisensäureester enthält. Der Rest ist im wesentlichen Diphenylcarbonat.

Die Gesamtausbeute beträgt also 1442 g (86,6% der theoretischen Ausbeute), zuzüglich 42,5 g (5,1% der theoretischen Ausbeute) im Rückstand.

Beispiel 8

Man arbeitet wie im Beispiel 7a), leitet das $COCl_2$ jedoch bei 110°C ein.

Nach einer Phosgenierzeit von 13 Stunden waren 530 g $COCl_2$ verbraucht.

Durch Destillation erhält man 745 g Chlorameisensäurephenylester (Gehalt 99,8%; Ausbeute 89,3% der theoretischen Ausbeute).

Es hinterbleibt ein Rückstand von 83,5 g, der noch 40% Chlorameisensäurephenylester enthält (33,4 g; 4,0% der theoretischen Ausbeute).

Beispiele 9–13

Beispiele mit weiteren Katalysatoren, die entsprechend Beispiel 4a) durchgeführt wurden, sind in der folgenden Tabelle zusammengefasst.

Tabelle

| Beispiel | Einsatz (g Phenol | Katalysator | Menge | Reakt.-temp. (°C) | Phosgen-Verbrauch (g) | Reakt.-zeit (h) | Ausbeute (g) im Destillat | im Rückstand | Ausbeute (%d.th.Ausbeute) im Destillat | im Rückstand | Gesamt |
|---|---|---|---|---|---|---|---|---|---|---|---|
| 9 | 250 | 1-Phenyl-2,5-dihydro-2-methylphospholoxid | 10,2 g | 120–130 | 300 | 17 | 370,8 | 3,6 | 89,1 | 0,9 | 90,0 |
| 10 | 500 | $C_6H_5$-$Cl_2$ | 34,1 g | 120–125 | 510 | 10 | 604,7 | 64,5 | 72,6 | 7,7 | 80,4 |
| 11 | 500 | $(C_6H_5)_3P(CH_3)Br$ | 39,1 g | 120–125 | 555 | 11 | 713,7 | 40,4 | 85,7 | 4,9 | 90,6 |
| 12 | 500 | $(n\text{-}C_4H_9)_3P$ | 21,5 g | 120–125 | 560 | 15 | 729,3 | 43,2 | 87,6 | 5,2 | 92,8 |
| 13 | 500 | $(C_6H_5)_3PCl_2$ | 35,5 g | 120–125 | 520 | 8,5 | 774,2 | 26,4 | 93,0 | 3,2 | 96,2 |

Beispiel 14

In einem 1 l Kolben, der mit Rührer, Gaseinleitungsrohr, Innenthermometer und Tiefkühl-Rückflusskühler ausgestattet ist, werden 385,7 g p-Chlorphenol (3,0 Mol) und 16 g Triphenylphosphin vorgelegt. Man erhitzt auf 125°C und leitet in 16 Stunden insgesamt 310 g $COCl_2$ so langsam ein, dass Phosgen gerade nicht am Rückfluss zu sieden beginnt. Nach Entfernen des Kühlers wird mit Stickstoff restliches Phosgen ausgetrieben, letzte Reste werden dann im Vakuum entfernt.

Man erhält rohen Chlorameisensäure-4-chlorphenylester, laut Amintitration in einer Ausbeute von 85% der theoretischen Ausbeute als blassgelbe Schmelze, die beim Abkühlen fest wird.

Reinen Ester erhält man durch Destillation [Kp. 104°C/13,3 mbar (= 1,33 kPa)].

Beispiel 15

In einer Apparatur, wie in Beispiel 1 beschrieben, legt man 324,4 g p-Kresol (3,0 Mol) und 15,7 g Triphenylphosphin vor, erhitzt auf 120 bis

125°C und leitet in 12 Stunden 330 g $COCl_2$ ein. Man rührt 1 Stunde nach, zieht im Vakuum überschüssiges Phosgen ab und destilliert 461,0 g Chlorameisensäure-4-tolylester ab, der laut Titration mit Di-n-Butylamin sowie laut gaschromatographischer Analyse praktisch rein ist [Kp. 92 bis 94°C/17,3 mbar (= 1,73 kPa)]. Die Ausbeute beträgt 90,1%; geringe Mengen finden sich noch im Destillationsrückstand.

Beispiel 16

In einer Apparatur, wie in Beispiel 1 beschrieben, legt man 433 g 2-Naphthol (3,0 Mol) und 15,7 g Triphenylphosphin vor. Man erhitzt auf 120 bis 125°C und leitet in 12 Stunden 325 g $COCl_2$ ein. Man rührt 1 Stunde nach, zieht im Vakuum überschüssiges Phosgen ab und erhält rohen Chlorameisensäure-2-naphthylester in einer Ausbeute von 75% der theoretischen Ausbeute.

Er kann durch Vakuumdestillation gereinigt werden [Kp. 136 bis 138°C/10 mbar (= 1,0 kPa)] bzw. 121 bis 124°C/2 mbar (= 0,2 kPa). Ein entsprechendes Ergebnis wird erhalten, wenn anstelle von 2-Naphthol das 1-Naphthol umgesetzt wird.

Beispiel 17

In eine Lösung von 114,0 g (0,5 Mol) 2,2-Bis-(4-hydroxyphenyl)-propan («Bisphenol A») und 5,0 g Triphenylphosphin in 1 l Chlorbenzol wird bei 120 bis 125°C innerhalb von 7 Stunden 125 g Phosgen so eingeleitet, dass an einem nachgeschalteten mit $CO_2$ gekühlten Rückflusskühler $COCl_2$ nicht oder nur schwach kondensiert. Dann wird 2 Stunden lang bei 115°C nachgerührt. Das Lösungsmittel wird am Rotationsverdampfer abdestilliert.

Man erhält 190,7 g 90,0%iges 2,2-Bis-(4-Chloroformoxyphenyl)-propan (97,2% der theoretischen Ausbeute); (Gehalt bestimmt durch Titration mit Di-n-butylamin).

Das Massenspektrum einer aus Leichtbenzin umkristallisierten Probe zeigt Signale bei m/e = 352/354 («Molekular-peak»), das NMR-Spektrum (in $CDCl_3$) zeigt Signale bei $\delta$ = 1,67 (s), 7,1 (d) und 7,3 (d) ppm.

Beispiel 18

In einer Apparatur, wie in Beispiel 1 beschrieben, legt man 500 g 2-sec.-Butyl-phenol (99%ig, $\triangleq$ 3,30 Mol) und 5,8 g Triphenylphosphin vor. Man erhitzt auf 140–145°C und leitet in 6,5 h 360 g Phosgen ein. Man rührt 1 Stunde nach und arbeitet auf wie in Beispiel 1 beschrieben.

Durch Destillation erhält man 669,5 g reinen, 100%igen Chlorameisensäure-2-sec.-butyl-phenylester vom Siedepunkt 116–118°C/21 mbar [Lit.: 71°/1,3 mbar (= 0,13 kPa); DE-OS 22 13 408), dessen Gehalt durch Amintitration ermittelt werden kann.

Das entspricht einer Ausbeute von 95,5% der theoretischen Ausbeute.

Es hinterbleibt ein Rückstand von 31 g, der noch ca. 50% seines Gewichtes an Produkt enthält. Der Katalysator verbleibt im Destillationsrückstand und kann mit diesem in einen folgenden Reaktionsansatz neu eingesetzt werden, ohne dass ein Aktivitätsverlust beobachtet wird.

**Patentansprüche**

1. Verfahren zur Herstellung aromatischer Chlorameisensäurearylester aus Phenolen und Phosgen, dadurch gekennzeichnet, dass die Umsetzung in homogener flüssiger Phase bei einer Temperatur von 60 bis 180°C in Gegenwart organischer Phosphorverbindungen der Formel

$R^1R^2R^3PR^4{}_nX_n$

in der
$R^1$, $R^2$ und $R^3$ unabhängig voneinander für Wasserstoff, Alkyl, Alkenyl, Aralkyl, Aryl oder homöopolar an Phosphor gebundenes Halogen stehen und wobei zwei der genannten Reste mit dem Phosphoratom einen 5- oder 6-gliedrigen phosphorhaltigen gesättigten oder ungesättigten Heterocyclus bilden können,
X für OH, homöopolar gebundenes Halogen oder ein anorganisches oder organisches Säureanion steht,
$R^4$ Wasserstoff oder Alkyl bedeutet oder, wenn X Halogen bedeutet, auch Halogen bedeuten kann,
n 0 oder 1 bedeutet,
und in der weiterhin $R^4$ und X gemeinsam für Sauerstoff oder Schwefel stehen können, wobei die organische Phosphorverbindung jedoch maximal zwei Halogenatome enthält, durchgeführt wird.

2. Verfahren nach Anspruch 1, dadurch gekennzeichnet, dass als organische Phosphorverbindung eine Verbindung der Formel

$R^5R^6R^7PR^8{}_nY_n$

in der
$R^5$, $R^6$ und $R^7$ unabhängig voneinander für Alkyl, Benzyl, Phenyl oder homöopolar an Phosphor gebundenes Chlor stehen,
Y für OH, Chlor oder Brom steht,
$R^8$ Wasserstoff oder Alkyl bedeutet oder wenn Y Chlor oder Brom ist, auch Chlor oder Brom bedeuten kann,
n die obengenannte Bedeutung hat und
in der weiterhin $R^8$ und Y gemeinsam für Sauerstoff stehen können, wobei die organische Phosphorverbindung jedoch maximal zwei Chlor- bzw. Bromatome enthält, eingesetzt wird.

3. Verfahren nach Anspruch 1, dadurch gekennzeichnet, dass als organische Phosphorverbindung Triphenylphosphin, Triphenylphosphinoxid, Tributylphosphin, Tributylphosphinoxid, Triphenylphosphindichlorid oder Tributylphosphindichlorid eingesetzt wird.

4. Verfahren nach Anspruch 1 bis 3, dadurch gekennzeichnet, dass die organische Phosphorverbindung in einer Menge von 0,1 bis 20 Mol-% pro Äquivalent organische Hydroxylgruppe eingesetzt wird.

5. Verfahren nach Anspruch 1 bis 4, dadurch gekennzeichnet, dass die organische Phosphorverbindung zu wiederholten Malen eingesetzt wird.

6. Verfahren nach Anspruch 1 und 5, dadurch gekennzeichnet, dass zum wiederhotlen Einsatz der organischen Phosphorverbindung der diese Phosphorverbindung enthaltende Destillationsrückstand ohne weitere Aufarbeitung eingesetzt wird.

7. Verfahren nach Anspruch 1, dadurch gekennzeichnet, dass man bei einer Temperatur zwischen 80 und 160°C arbeitet.

8. Verfahren nach Anspruch 1, dadurch gekennzeichnet, dass als homogene flüssige Phase die Lösung des Phenols in einem inerten Lösungsmittel eingesetzt wird.

**Claims**

1. Process for the preparation of aromatic chloroformic acid aryl esters from phenols and phosgene, characterised in that the reaction is carried out in a homogeneous liquid phase at a temperature of 60 to 180°C in the presence of organic phosphorus compounds of the formula

$R^1R^2R^3PR^4{}_nX_n$

in which
$R^1$, $R^2$ and $R^3$ independently of one another represent hydrogen, alkyl, alkenyl, aralkyl, aryl or halogen which is bonded by a homopolar bond to phosphorus and two of the said radicals together with the phosphorus atom can form a 5-membered or 6-membered phosphorus-containing saturated or unsaturated heterocyclic radical,
X represents OH, homopolar-bonded halogen or an inorganic or organic acid anion,
$R^4$ denotes hydrogen or alkyl or, if X denotes halogen, can also denote halogen and
n denotes 0 or 1,
and in which, furthermore,
$R^4$ and X together can represent oxygen or sulphur, the organic phosphorus compound containing, however, at most two halogen atoms.

2. Process according to Claim 1, characterised in that the organic phosphorus compound used is a compound of the formula

$R^5R^6R^7PR^8{}_nY_n$

in which
$R^5$, $R^6$ and $R^7$ independently of one another represent alkyl, benzyl, phenyl or chlorine which is bonded by a homopolar bond to phosphorus,
Y represents OH, chlorine or bromine,
$R^8$ denotes hydrogen or alkyl or, if Y is chlorine or bromine, can also denote chlorine or bromine,
n has the abovementioned meaning,
and in which, furthermore,
$R^8$ and Y together can represent oxygen, the organic phosphorus compound containing, however, at most the chlorine or bromine atoms.

3. Process according to Claim 1, characterised in that the organic phosphorus compound used is triphenylphosphine, triphenylphosphine oxide, tributylphosphine, tributylphosphine oxide, triphenylphosphine dichloride or tributylphosphine dichloride.

4. Process accoriding to Claim 1 to 3, characterised in that the organic phosphorus compound is employed in an amount of 0.1 to 20 mol % per equivalent of organic hydroxyl grup.

5. Process according to Claim 1 to 4, characterised in that the orgnaic phosphorus compound is re-used several times.

6. Process according to Claim 1 and 5, characterised in that for re-use of the organic phosphorus compound the distillation residue containing this phosphorus compound is used without further working up.

7. Process according to Claim 1, characterised in that the reaction is carried out at a temperature between 80 and 160°C.

8. Process according to Claim 1, characterised in that the homogeneous liquid phase used is the solution of the phenol in an inert solvent.

**Revendications**

1. Procédé de préparation d'esters aryliques aromatiques de l'acide chloroformique à partir de phénols et du phosgène, caractérisé en ce que l'on effectue la réaction en phase liquide homogène à une température de 60 à 180°C en présence de composés organiques du phosphore de formule:

$R^1R^2R^3PR^4{}_nX_n$

dans laquelle
$R^1$, $R^2$ et $R^3$ représentent chacun, indépendamment les uns des autres, l'hydrogène, un groupe alkyle, alcényle, aralkyle, aryle ou un halogène relié au phosphore par une liaison homopolaire, deux des restes mentionnés pouvant former avec l'atome de phosphore un hétérocycle phosphoré saturé ou insaturé à 5 ou 6 chaînons,
X représente OH, un halogène à liaison homopolaire ou un anion d'acide organique ou minéral,
$R^4$ représente l'hydrogène ou un groupe alkyle ou bien encore, lorsque X représente un halogène, un halogène,
n est égal à 0 ou 1,
et dans laquelle, en outre, $R^4$ et X peuvent représenter ensemble l'oxygène ou le soufre, le composé organique du phosphore, toutefois contenant au maximum deux atomes d'halogènes.

2. Procédé selon la revendication 1, caractérisé en ce que l'on utilise en tant que composé organique du phosphore un composé de formule:

$R^5R^6R^7PR^8{}_nY_n$

dans laquelle
$R^5$, $R^6$ et $R^7$ représentent chacun, indépendamment les uns des autres, un groupe alkyle, benzyl, phényle ou un atome de chlore relié au phosphore par une liaison homopolaire,
Y représente OH, le chlore ou le brome,
$R^8$ représente l'hydrogène ou un groupe alkyle ou

bien encore, lorsque Y représente le chlore ou le brome, lui-même le chlore ou le brome,
n a la signification indiquée ci-dessus, et
dans laquelle, en outre, $R^8$ et Y peuvent représenter en commun l'oxygène, ce composé organique du phosphore, toutefois, contenant au maximum deux atomes de chlore ou de brome.

3. Procédé selon la revendication 1, caractérisé en ce que l'on utilise en tant que composé organique du phosphore la triphénylphosphine, l'oxyde de tirphénylphosphine, la tributylphosphine, l'oxyde de tributylphosphine, le dichlorure de la triphénylphosphine ou le dichlorure de la tributylphosphine.

4. Procédé selon les revendications 1 à 3, caractérisé en ce qu'on utilise le composé organique du phosphore en quantité de 0,1 à 20 moles-% par équivalent de groupe hydroxy organique.

5. Procédé selon les revendications 1 à 4, caractérisé en ce que le composé organique du phosphore est réutilisé à plusieurs reprises.

6. Procédé selon les revendications 1 et 5, caractérisé en ce que, pour l'utilisation répétée du composé organique du phosphore, on utilise le résidu de distillation contenant ce composé du phosphore, sans autre traitement.

7. Procédé selon la revendication 1, caractérisé en ce que l'on opère à une température de 80 à 160°C.

8. Procédé selon la revendication 1, caractérisé en ce que la phase liquide homogène est la solution du phénol dans un solvant inerte.